(19) Europäisches Patentamt
European Patent Office
Office européen des brevets

(11) Numéro de publication: **0 308 564 B1**

(12) **FASCICULE DE BREVET EUROPEEN**

(45) Date de publication de fascicule du brevet: **22.07.92**

(51) Int. Cl.$^5$: **A61K 31/14**

(21) Numéro de dépôt: **87402632.1**

(22) Date de dépôt: **23.11.87**

Le dossier contient des informations techniques présentées postérieurement au dépôt de la demande et ne figurant pas dans le présent fascicule.

(54) **Composition inhibitrice ou destructrice d'au moins un être vivant unicellulaire renfermant un fluorure d'ammononium quaternaire et procédé de préparation de ce sel.**

(30) Priorité: **23.09.87 FR 8713156**

(43) Date de publication de la demande:
**29.03.89 Bulletin 89/13**

(45) Mention de la délivrance du brevet:
**22.07.92 Bulletin 92/30**

(84) Etats contractants désignés:
**AT BE CH DE ES FR GB GR IT LI LU NL SE**

(56) Documents cités:
**EP-A- 0 253 037**
**WO-A-80/00057**
**FR-A- 1 297 708**

**Japanese Abstracts, vol. 11, no.80, C-409, (2537), 11.03.87 & Jp-A-60-73227**

(73) Titulaire: **ATLANTIC PHARMACEUTICAL PRODUCTS LIMITED**
**Celbridge**
**Dunaghcumper Co Kildare(IE)**

(72) Inventeur: **Bourbon, Pierre**
**36, rue Volta,**
**31000 Toulouse(FR)**
Inventeur: **Billot, Pierre**
**20, Boulevard de la Saussaye**
**92200 Neuilly sur Seine(FR)**
Inventeur: **Lagny, Pierre**
**6, Castle Town Court**
**Celbridge, c/o Kildare(IE)**

(74) Mandataire: **Lassiaille, Christian Michel**
**Bouju Derambure (Bugnion) S.A. 4, Square Jean Moulin**
**F-73100 Aix les Bains(FR)**

**EP 0 308 564 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention concerne l'inhibition ou la destruction des êtres vivants uncellulaires tels que les protozoaires, microbes, bactéries, gamètes, champignons, levures ou autres et des virus. Elle vise plus particulièrement les domaines techniques de la contraception locale, de l'antibiothérapie, de l'antiseptie, de la lutte contre les maladies sexuellement transmissibles y compris les mycoses, de la lutte contre les affections virales, et ce dans le cadre de la pharmacie à usage humain ou vétérinaire ou de la cosmétique. L'invention peut également avantageusement être appliquée dans le domaine de la désinfection de surfaces ou encore en agriculture par exemple dans la lutte contre les fongus et les bactéries.

On connait déjà de nombreuses substances inhibant ou détruisant les êtres vivants unicellulaires, et parmi elles les agents tensio-actifs tels que les sels d' ammonium quaternaire. En particulier on sait que les halogénures d'ammonium quaternaire tels que le chlorure de benzalkonium (ou chlorure de diméthyl benzyl ammonium), seuls ou en combinaison avec d'autres principes actifs, sont avantageux dans ces applications(voir par exemple brevets anglais 1 554 615, français 2 431 859, 2 483 177, 2 379 508, 2 384 497, 2 457 641, 2 573 624, 2 418 221, 2 562 888, européens 0 243 713, 0 175 338, 0 132 963, 0 127 131, 0 094 562, 0 076 136, 0 068 399, 0 037 593, demande de brevet européen non publiée n° 86 402 716.4 déposée le 8.12.1986 et demandes internationales WO 84/00877 et WO 84/02649).

On sait également par ailleurs que les halogénures d'ammonium quaternaire les plus faciles à fabriquer et les mieux connus, pour ces applications et dans d'autres applications connues, sont les chlorures, les iodures, le bromures et les chloroiodites (voir brevets mentionnés ci-dessus, et également brevets français 2 002 945, 2 185 717, 2 267 092, 2 366 260, 2 366 261, 2 391 991, 2 418 220, 2419 024, 2 482 090, 2 505 325, 2 517 672, 2562 799 et européens 0 191 236).

Par ailleurs, l'art antérieur a déjà fourni de nombreux procédés de fabrication de ces sels d'ammonium quaternaires, mais qui ne sont applicables directement, parmi les halogènures d'ammonium quaternaires qu'aux chlorures et/ou iodures, et/ou bromures, et/ou chloroiodites. (voir brevets ci-dessus et également brevets français 2 472 558, 2 033 044 et européens 0 094 552 et 0 012 296).

On connait également le fluorure d'ammonium et ses procédés de préparation et purification (voir par exemple brevets français 2 244 713, 2 253 710 et européen 0 002 016), et des sels d'ammonium quaternaires perfluorés ou polyfluorés (par exemple brevets français 2 038 421, 2 051 095, 2 153 489 et européens 0 073 760, 0 100 478, 0 100 477, 0149 172).

Le fluor ionique est par ailleurs bien connu pour ses propriétés anti-caries dans les applications dentaires (par exemple brevet américain 4 473 547; dictionnaire VIDAL, 1985, page 582, O.V.P., Paris, "fluor monal"), éventuellement associé à un ammonium quaternaire cationique (voir brevets français 1 486 676 et 1 297 708). Dans ces derniers documents on met en évidence l'avantage d'associer dans un même composé d'une part le fluor bien connu pour ses propriétés anti-caries, et d'autre part les sels d' ammonium quaternaire tensio-actifs connus pour leurs propriétés bactéricides. Cependant aucune activité véritablement synergique n'est démontrée dans ces applications pour les dentifrices, avec les fluorures d'ammonium quaternaire testés que ce soit vis-à-vis des propriétés anti-caries ou vis-à-vis des propriétés bactéricides.

Le brevet français 1 297 708 décrit en particulier le fluorure de lauryl-benzyl-diméthyl-ammonium, un procédé de préparation de ce fluorure et son application dans une pâte dentifrice.

Enfin, le brevet italien 1 153 530 décrit un procédé de préparation de carbonates d'ammonium quaternaires puis, à partir de ces carbonates, des halogénures d'ammonium quaternaires par échange d'anions avec l'acide correspondant plus fort que l'acide carbonique. Cependant ce brevet ne mentionne pas comment ce procédé peut être appliqué à la préparation de fluorures. Par ailleurs si les composés obtenus grâce à ce procédé sont aptes à être appliqués dans la désinfection de surfaces et dans demande de brevet européen non publiée n° 86 402 716.4.

L'invention concerne donc l'application d'au moins un fluorure d'ammonium quaternaire purifié de formule générale telle qu'indiquée ci-dessus où R est un radical alcoyle pouvant varier entre $C_8$ $H_{17}$ et $C_{18}$ $H_{37}$ pour obtenir une composition destinée à inhiber ou à détruire au moins un virus ou un être vivant unicellulaire tel qu'un protozoaire, microbe, bactérie, gamète, champignon, levure ou autre en dehors de la sphère dentaire.

L'invention concerne également l'application de ce même fluorure d'ammonium quaternaire purifié pour obtenir une composition virucide, ou une composition fongicide, ou une composition pharmaceutique bactéricide destinée à être appliquée en dehors de la sphère dentaire, ou une composition destinée à être appliquée sur les organes génitaux pour lutter contre les maladies sexuellement transmissibles, ou pour obtenir une composition contraceptive locale, notamment spermicide, ou pour obtenir une composition cosmétique ou d'hygiène corporelle destinée à être appliquée en dehors de la sphère dentaire, ou pour

obtenir une composition pour la désinfection locale du corps humain ou de surfaces en dehors de la sphère dentaire, ou pour obtenir une composition pharmaceutique antibiotique administrable par voie générale, ou pour obtenir une composition topique antibiotique, ou pour obtenir une composition topique destinée à lutter contre les affections virales, notamment les affections virales de la peau et des organes génitaux, ou pour obtenir une composition pharmaceutique administrable par voie générale ou parentérale destinée à lutter contre les virus ou les rétrovirus, notamment l'herpès ou le LVA (ou HIV) responsable du SIDA, ou pour obtenir une composition destinée à lutter contre les affections mycosiques.

Selon l'invention, R est compris entre $C_{12}H_{25}$ et $C_{14}H_{29}$. On utilise entre 0,05 % et 7 % en poids, notamment de l'ordre de 1 %, d'un tel sel d'ammonium quaternaire purifié. Selon l'invention, on utilise un autre principe actif choisi en fonction de la destination de la composition -notamment un antibiotique- en combinaison avec le fluorure d'ammonium quaternaire. Et, selon l'invention, on utilise en outre un fluorure métallique. Selon l'invention, on utilise du fluorure de Lithium. Et, selon l'invention, on utilise en outre le conservateur kathon (marque déposée).

L'invention concerne également l'utilisation d'un même fluorure d'ammonium quaternaire à titre de contraceptif local, notamment de spermicide.

L'invention concerne également une composition inhibitrice ou destructrice d'au moins un virus ou d'au moins un être vivant unicellulaire tel qu'un protozoaire, microbe, bactérie, gamète, champignon, levure ou autre, caractérisée en ce qu'elle renferme au moins un tel fluorure d'ammonium quaternaire. Selon l'invention, R est compris entre $C_{12}H_{25}$ et $C_{14}H_{29}$. Selon l'invention, la composition comporte entre 0,05 % et 7 % en poids, notamment de l'ordre de 1 %, d'un tel sel d'ammonium quaternaire suffisamment purifié de façon à pouvoir être utilisée pour l'être humain ou animal.

L'invention concerne enfin une composition antibiotique caractérisée en ce qu'elle renferme un principe antibiotique et un tel fluorure d'ammonium quaternaire. Selon l'invention, R est compris entre $C_{12}H_{25}$ et $C_{14}H_{29}$.

Exemple de préparation d'un fluorure de benzalkonium en $C_{12}$ :

Trois kilogrammes de $C_{12}$ diméthyl-benzyl-ammonium méthylcarbonate sont traités par 6,31 de HF 1,25N solubilisé dans le méthanol.

Il se produit un fort dégagement d'anhydride carbonique. En fin de réaction le pH est compris entre 5 et 6. Après évaporation sous vide du méthanol, on obtient une masse pâteuse translucide jaune ambré.

La purification de cette masse pâteuse est effectuée par dissolution à chaud de la masse translucide dans l'acétone à chaud. Après refroidissement lent, on sépare le résidu pâteux qui est ensuite débarrassé du solvant restant sous vide.

La pureté du fluorure de benzalkonium obtenu est contrôlée par un dosage de l'ion $F^-$ à l'électrode spécifique, et un dosage HPLC (chromatographie liquide haute pression) de l'ammonium quaternaire. Dans cet exemple, la pureté du produit obtenu a été de 99 %.

Exemple de préparation d'un fluorure de benzalkonium en $C_{12}$ avec purification préalable :

Un kilogramme de $C_{12}$ diméthyl-benzyl-ammonium méthylcarbonate porté à son point de fusion est lavé à l'éther de pétrole et séché sous vide.

Il est ensuite attaqué à l'état fondu par 2,761 de HF 1,5N dans le méthanol. Il se produit un fort dégagement d'anhydride carbonique. En fin de réaction le pH est compris entre 5 et 6. Après évaporation sous vide du méthanol, on obtient une masse pâteuse translucide jaune ambré. La purification est effectuée par dissolution à chaud de la masse translucide dans l'acétone à chaud. Après refroidissement lent, on sépare le résidu pâteux qui est ensuite débarrassé du solvant restant sous vide.

La pureté du produit contrôlée selon la même méthode que dans l'exemple précédent a été de 99%.

Exemple de préparation d'un fluorure de benzalkonium en $C_{14}$ :

Les mêmes procédé de fabrication que dans les 2 exemples précédents ont été utilisés un substituant au $C_{12}$ diméthyl-benzyl-ammonium méthylcarbonate, le $C_{14}$ diméthyl-benzyl-ammonium méthylcarbonate.

Le fluorure de benzalkonium obtenu se présente également sous forme d'une masse pâteuse jaune ambré.

Après purification, la pureté a été respectivement de 98% et 99%.

Les fluorures de benzalkonium préparés comme indiqués précédemment ont été utilisés dans les essais décrits ci-dessous, permettant de mesurer leur activité.

Sauf indication contraire, les méthodologies emmployées ont été celles déjà décrites dans la demande de brevet européen 86 402 416.4 déposée le 8.12.1986. L'enseignement de cette demande de brevet européen est considéré comme connu dans la description ci-après.

Essai n° 1 : Activité spermicide des compositions in-vitro :

Les tests de SANDERS-CRAMER selon les normes de l'IPPF (Fédération Internationale Pour le Planning Familial) ont été réalisés pour déterminer la concentration minimale inhibitrice du fluorure de benzalkonium. On a ainsi obtenu une concentration minimale inhibitrice (CMI) du fluorure de benzalkonium en $C_{12}$ de 2 mg/l, soit 0,002%. Cette valeur est à comparer à la CMI du chlorure de benzalkonium seul qui est de 0,063% et à la CMI du chlorure de benzalkonium en présence d'anion $F^-$ à 1 µg/ml, qui est de 0,003%. Comme on le constate donc, le fluorure de benzalkonium procure une activité supérieure au chlorure de benzalkonium seul, mais également supérieure à l'association du chlorure de benzalkonium à l'anion fluorure $F^-$ par exemple sous forme de fluorure de sodium.

Essai n° 2 : Activités comparées des préparations galéniques spermicides:

Cet essai in-vitro a été réalisé sur des préparations galéniques renfermant comme principe actif soit du chlorure de benzalkonium seul, soit du chlorure de benzalkonium avec 0,45% en poids d'anion fluorure $F^-$ sous forme de fluorure de sodium, soit du fluorure de benzalkonium. Les CMI sont déterminées après simulation in-vitro des conditions réelles in-vivo (extraction, solubilisation, ...). Le test de SANDERS-CRAMER de l'IPPF est ensuite réalisé in-vitro. Les résultats obtenus sont résumés dans les tableaux suivants :

| FORME GALENIQUE | Proportions de chlorure de benzalkonium seul (en poids) dans la forme galénique | CMI (% en poids) du chlorure de benzalkonium |
|---|---|---|
| Ovule | 1,2% | 0,0063% |
| Crème | 1,2% | 0,0083% |
| Tampon | 1,2% | 0,0075% |
| Comprimé | 3,12% | 0,0095% |
| Film soluble | 1,2% | 0,0080% |
| Gelée | 1,2% | 0,0080% |

| FORME GALENIQUE | Proportions de chlorure de benzalkonium seul (en poids) dans la forme galénique avec 0,45% d'anion $F^-$ | CMI (% en poids) du chlorure de benzalkonium |
|---|---|---|
| Ovule | 1,2% | 0,0023% |
| Crème | 1,2% | 0,0030% |
| Tampon | 1,2% | 0,0025% |
| Comprimé | 3,12% | 0,0025% |
| Film soluble | 1,12% | 0,0017% |
| Gelée | 1,12% | 0,0033% |

| FORME GALENIQUE | Proportions de fluorore de benzalkonium (en poids) dans la forme galénique | CMI (% en poids) du fluorure de benzalkonium |
|---|---|---|
| Ovule | 1% | 0,0019% |
| Crème | 1% | 0,0022% |
| Tampon | 1% | 0,0020% |
| Comprimé | 2,5% | 0,0020% |
| Film soluble | 1% | 0,0012% |
| Gelée | 1% | 0,0026% |

On constate donc que le fluorure de benzalkonium a une concentration minimale inhibitrice très

inférieure à celle du chlorure de benzalkonium et même à celle du chlorure de benzalkonium en présence d'anion $F^-$. Un effet synergique surprenant est donc dû au fluorure de benzalkonium.

Essai n° 3 : Activités fongicides comparées :

Les activités fongicides ont été testées sur trois souches de moisissure selon la norme AFNOR T72-201. Les trois souches de moisissure ont été les suivantes :

- Aspergillus versicolor CNCM 1187-79,
- Cladosporium cladosporioides CNCM 1185-79,
- Penicillium virrucosum var. cylopium CNCM 1186-79.

L'essai a été également réalisé sur une souche de levure : candida albicans (ATCC 2091) CNCM 1180-79.

Les essais ont été réalisés avec un temps de contact de 15mm à 20°C.

Deux types d'essais ont été réalisés : un premier essai comparant l'activité fongicide du chlorure de benzalkonium seul au fluorure de benzalkonium seul, et un deuxième essai comparant l'activité fongicide du chlorure de benzalkonium associé au fluorure de lithium et au conservateur KATHON®, l activité fongicide du fluorure de benzalkonium associée au fluorure de lithium et au même conservateur KATHON®.

Les résultats sont résumés dans les tableaux suivants :

| | Concentration fongicide selon norme AFNOR en µg/ml (ppm) | |
|---|---|---|
| | Chlorure de benzalkonium | Fluorure de benzalkonium |
| Aspergillus versicolor | 100 | 100 |
| Cladosporium | 200 | 200 |
| Penicillium | 50 | 50 |
| Candida Albicans | 50 | 50 |

| | Concentration fongicide selon norme AFNOR en µg/ml (ppm) | |
|---|---|---|
| | Chlorure de benzalkonium + FLI 0,9µg/ml + Kathon 0,49µg/ml | Fluorure de benzalkonium + FLI 0,9µg/ml + Kathon® 0,49µg/ml |
| Aspergillus versicolor | 100 | 100 |
| Cladosporium | 2000 | 100 |
| Penicillium | 50 | 50 |
| Candida Albicans | 50 | 50 |

On constate donc que l'association du fluorure de benzalkonium avec le fluorure de lithium et le conservateur Kathon® est dans certains cas meilleure que l'association du chlorure de benzalkonium au fluorure de lithium et au même conservateur Kathon®.

Essai n° 4 : Activité sur les êtres pathogènes responsables des maladies sexuellement transmissibles :

Les essais ont été réalisés sur des souches de Gonocoque, Trichomonas, Chlamydia, Gardnerella, et Bacille de Ducrey. Les résultats obtenus sont sont résumés dans le tableau suivant :

| Souches : | Concentration minimale inhibitrice (C.M.I.) Chlorure de benzalkonium seul | Concentration minimale inhibitrice (C.M.I.) Chlorure de benzalkonium + $F^-$ 1 microgramme/ml | Concentration minimale inhibitrice (C.M.I) Fluorure de benzalkonium |
|---|---|---|---|
| Gonocoque | 1,15mg/litre | 0,60mg/litre | 0,44mg/litre |
| Thrichomonas | 1,3mg/litre | 0,9mg/litre | 0,7mg/litre |
| Chlamydia | 100mg/litre | 85mg/litre | 69mg/litre |
| Gardnerella | 50mg/litre | 41mg/litre | 32,4mg/litre |
| Bacille de Ducrey | 75mg/litre | 62mg/litre | 50,5mg/litre |

On constate donc une très forte diminution de la concentration minimale inhibitrice pour le fluorure de benzalkonium.

Essai n° 5 : Activité bactéricide sur des souches normalisées :

Cet essai a été mené selon la norme AFNOR NFT 72-150, Mars 1981. Les résultats obtenus sont résumés dans le tableau suivant :

| Souches : | Concentration minimale inhibitrice (C.M.I.) Chlorure de benzalkonium | Concentration minimale inhibitrice (C.M.I.) Chlorure de benzalkonium + $F^-$ 1 microgramme/ml | Concentration minimale inhibitrice (C.M.I) Fluorure de benzalkonium |
|---|---|---|---|
| Pseudomonas aeruginosa CNCM A 22 | 31,25mg/litre | 18mg/litre | 14,2mg/litre |
| Escherichia coli CNCM 54 127 | 6,57mg/litre | 3mg/litre | 2,5mg/litre |
| Staphyloccocus aureus Souche Oxforf CNCM 53 154 | 1,56mg/litre | 1,1mg/litre | 0,9mg/litre |
| Staphyloccocus faecalis CNCM 5 855 | 4mg/litre | 3,6mg/litre | 2,9mg/litre |
| Mycobacterium smegmatis CNCM 7 326 | 30mg/litre | 26mg/litre | 21,9mg/litre |

Là encore on constate une nette amélioration pour le fluorure de benzalkonium.

Essai n° 6 : Activité virucide :

Cet essai a été réalisé selon la méthode de filtration sur gel de SEPHADEX® LH 20.

Les antiseptiques utilisés ont été le chlorure de benzalkonium $C_{14}$ en poudre, le fluorure de benzalkonium $C_{12}$ en cristaux, le fluorure de lithium en poudre. Les gammes de dilution de raison géométrique 2 dans l'eau bidistillée stérile ont été réalisées, et les essais sont effectués pour 10, 30, 50,80, 100, 200, 500mg/litre de chlorure de benzalkonium et de fluorure de benzalkonium, avec ou sans adjonction de fluor de lithium à 1 ou 1,70mg/litre.

Les cultures cellulaires employées sont des cellules VERO (souches ATCC CCL81) entretenues sur milieu DMEM (EUROBIO®) additionnées de 5% de sérum de veau foetal et d'antiobiotiques (streptomycine à 50 microgramme par millilitre et penicilline à 200.000 $\mu$/l).

Le virus étudié a été le polio virus SABIN® type I. Il est cultivé sur cellules VERO en boîte de ROUX. La détermination du titre infectieux a été réalisée selon la méthode de REED et MUENCH, avec l'aide de la table de conversion de WYSHAK et DETRE.

L'essai a été mené en trois étapes, avec tout d'abord la détermination du seuil de cytotoxicité des antiseptiques, puis l'étude de la capacité des cellules traitées par les antiseptiques à développer l'infection et enfin la détermination des effets virucides de ces antiseptiques.

1- Détermination du seuil de cytotoxicité des antiseptiques :

Avant toute recherche des effets virucides, il faut rechercher la dilution d'antiseptiques responsables d'une activité cytotoxique, et cela avant et après la filtration.

Avant la filtration, les cellules VERO sont cultivées sur des microplaques de filtration à 96 puits. Les antiseptiques sont alors déposés en dilution croissante de raison géométrique à raison de 0,1ml par capsule et de 8 capsules par dilution sur les tapis cellulaires. Les cellules et les dilutions de substances virucides sont laissées en contact une heure à 37° C puis les substances antiseptiques sont éliminées et remplacées par le milieu de culture sans sérum. Il est alors possible de calculer la DLO (dilution d'antiseptiques n'altérant pas les cellules cultivées) après une incubation de 48 heures.

Après la filtration, dans l'appareillage de filtration on introduit 30ml de la suspension de gel SEPHADEX® LH 20. Cette opération est suivie d'une centrifugation, puis la solution d'antiseptique déposée à raison de 2ml sur la colonne de SEPHADEX®. Une nouvelle centrifugation est réalisée avant la détermination de la DLO.

2- Capacité des cellules traitées par antiseptique à développer l'infection virale :

Les cellules VERO sont cultivées sur des microplaques à 96 puits. L'antiseptique est employé à sa DLO et laissé en contact pendant une heure puis remplacé par des dilutions croissantes de raison 10 de Poliovirus. Après contact d'une heure, la suspension virale est éliminée et remplacée par un milieu de culture sans sérum. la mesure de l'effet cytopathique se fait après une incubation de 48 heures à 37° C.

3- Détermination de l'activité virucide des antiseptiques :

Toutes les doses des antiseptiques utilisés sont bien évidemmnt inférieures au seuil de cytotoxicité enregistré précédemment. dans les mélanges contenant un volume égal de suspension virale et d'antiseptique, on prélève 1ml à 30, 60, 120 et 180 minutes. les prélèvements sont ensuite déposés sur les colonnes de SEPHADEX® puis centrifugés. Parallèlement, une supension témoin virale sans antiseptique est pratiquée dans les mêmes conditions.

Les résultats sont donnés par les tableaux suivants :

CBK : Chlorure de benzalkonium
FBK : Fluorure de benzalkonium
FLI : Fluorure de lithium

| Etude de seuil de cytotoxicité (DLO) | | |
|---|---|---|
| Antiseptique | Avant filtration | Après filtration |
| CBK | 0,05% | ~ 0,01% |
| CBK + FLI | 0,05% | ~ 0,01% |
| FBK | 0,01% | ~ 0,005% |
| FBK + FLI | 0,01% | ~ 0,005% |

| Capacité des cellules à développer l'infection virale | | | | |
|---|---|---|---|---|
| | Titre des suspensions virales (UFP/ml) | | | |
| Témoin virus $10^{7,94}$ | CBK + virus $10^{7,84}$ | [CBK + FLI] + virus $10^{7,84}$ | FBK + virus $10^{6,84}$ | [FBK + FLI] + virus $10^{6,04}$ |
| UFP = Unité Formant Plage (UFP/ml) | | | | |

| Activité virucide des antiseptiques sur le virus poliomiélitique après filtration sur gel, en fonction du temps de concentration | | | | | |
|---|---|---|---|---|---|
| Antiseptique | Concentration finale (en mg/l) | Titre viral exprimé en UFP/ml Temps de contact virus/antiseptique | | | |
| | | 30mn. | 60mn. | 120mn. | 180mn. |
| Chlorure de benzalkonium | 200 | $10^{1,74}$ | $10^{1,74}$ | $10^{1,74}$ | $10^{1\ 6\ 4}$ |
| | 100 | 0 | 0 | 0 | 0 |
| | 50 | $10^{1,74}$ | $10^{1,74}$ | $10^{1,64}$ | 0 |
| | 20 | $10^{1,84}$ | $10^{1,84}$ | $10^{1,74}$ | 0 |
| | 10 | $10^{4,84}$ | $10^{3,84}$ | $10^{3,84}$ | ~0 |
| Chlorure de benzalkonium + Fluorure de lithium (1mg/l) | 200 | $10^{1,84}$ | $10^{1,84}$ | $10^{1,74}$ | $10^{1,74}$ |
| | 100 | 0 | 0 | 0 | 0 |
| | 50 | $10^{1,84}$ | $10^{1,84}$ | $10^{1,84}$ | 0 |
| | 20 | $10^{1,74}$ | $10^{1,84}$ | $10^{1,64}$ | 0 |
| | 10 | $10^{3,84}$ | $10^{3,84}$ | $10^{3}$ | 0 |
| Fluorure de benzalkonium | 200 | $10^{1,74}$ | $10^{1,74}$ | $10^{1,64}$ | $10^{1,34}$ |
| | 100 | 0 | 0 | 0 | 0 |
| | 50 | $10^{1,34}$ | $10^{1,34}$ | 0 | 0 |
| | 20 | $10^{1,64}$ | $10^{1,64}$ | $10^{1,64}$ | 0 |
| | 10 | $10^{2,54}$ | $10^{2,54}$ | $10^{2,34}$ | $10^{2,34}$ |
| Fluorure de benzalkonium + Fluorure de lithium (1mg/l) | 200 | $10^{1,74}$ | $10^{1,74}$ | $10^{1,84}$ | $10^{1,84}$ |
| | 100 | 0 | 0 | 0 | 0 |
| | 50 | $10^{1,3}$ | $10^{1,3}$ | 0 | 0 |
| | 20 | $10^{1,84}$ | $10^{1,74}$ | $10^{1,34}$ | 0 |
| | 10 | $10^{3,84}$ | $10^{3,84}$ | $10^{3}$ | $10^{8,54}$ |

L'activité virucide en fonction du temps de contact virus-antiseptique montre donc que pour toutes les concentrations employées, le résultat obtenu, c'est-à-dire l'inhibation virale, est égale ou supérieure à celle exigée par l'Agence pour la Protection de l'Environnement (voir SS.BLOCK."Desinfection, sterilization and preservation "Editions Lea et Febiger, Philadelphia U.S.A., 1977).

Le chlorure de benzalkonium est actif à 0,01% en trente minutes de contact. Il en est de même avec le fluorure de benzalkonium. Cependant, pour des concentrations plus faibles, telles que 0,85% et 0,82%, l'inhibition virale est notée dès 120 minutes pour le fluorure de benzalkonium, alors qu'il faut attendre au moins 180 minutes pour le chlorure de benzalkonium.

Essai n° 7 : Activité virucide selon la norme AFNOR :

L'activité virucide du fluorure de benzalkonium a été déterminée en suivant la norme AFNOR T72-180. Les résultats suivants one été obtenus :

| 1/ Sur Herpès Simplex Virus Type 2 : | | |
|---|---|---|
| | Concentration inhibitrice (% en poids) | Toxicité cellulaire |
| Chlorure de benzalkonium | 0,01% | 0,05% |
| Fluorure de benzalkonium | 0,0075% | 0,08% |

| 2/ Sur Cytomégalovirus : | | |
|---|---|---|
| | Concentration inhibitrice (% en poids) | Toxicité cellulaire |
| Chlorure de benzalkonium | 0,01% | 0,05% |
| Fluorure de benzalkonium | 0,0075% | 0,08% |

LES MODES DE REALISATION PREFERENTIELS DE L'INVENTION POUR SES APPLICATIONS EN CONTRACEPTION LOCALE SONT LES SUIVANTES :

. OVULES :
Fluorure de benzalkonium 1,00%
Excipients : Glycérides semi synthétiques ou beurre de cacao, ou gélatine, glycérine et eau purifiée, antioxygènes, antiseptiques.

. CREMES :
Fluorure de benzalkonium 1,00%
Excipients : Eau distillée ou purifiée, humectants, gélifiants, émulsifiant, stabilisateur, antioxygène, antiseptique. (A répartir en proportions variables en fonction de la viscosité). pH compris entre 4,5 et 6,5 (acide citrique de préférence).

. ONGUENTS et POMMADES :
Fluorure de benzalkonium 1,00%
Excipients : Eau distillée ou purifiée, émulsifiant, excipients du type corps gras (vaseline, lanoline, lanovaseline, stéarovaseline) stabilisateur, antioxygène, antiseptique. (A répartir en proportions variables en fonction de la viscosité). pH compris entre 4,5 et 6,5 (acide citrique de préférence).

. GELEE :
Fluorure de benzalkonium 1,00%
Excipients : Dérivés solubles de la cellulose compatibles avec les tensio-actifs surfactifs cationiques, eau distillée ou purifiée, glycérine, sorbitol, antioxygène, antiseptique. (A répartir en proportions variables en fonction de la viscosité). pH compris entre 4,5 et 6,5 (acide citrique de préférence).

. FILM SOLUBLE :
Fluorure de benzalkonium 1,00%
Excipients : Alcool polyvinylique, glycérine, sorbitol, propylèneglycol, eau distillée ou purifiée antioxygène. pH compris entre 4,5 et 6,5 (acide citrique de préférence).

. COMPRIMES :
Fluorure de benzalkonium 20mg par comprimé
Excipients : Lactose, stéarate de magnésium, cellulose, amidon, acide citrique, bicarbonate de sodium.

. SAVONS SYNTHETIQUES EN PAINS OU EN PATES :
Fluorure de benzalkonium 2%
Excipients : Produits moussants et mouillants compatibles avec les ammonium quaternaire (par exemple tensio-actif amphotère du type bétaine ou amino-bétaine) émolliants, stabilisateur, antioxygène, antiseptique. pH compris entre 4,5 et 6,5 (acide citrique de préférence).

. SOLUTIONS PRETES A L'EMPLOI :

Fluorure de benzalkonium 0,40%

Excipients : Eau distillée ou purifiée, éthanol, antioxygène, glycérine, sorbitol, antiseptique. pH compris entre 4,5 et 6,5 (acide citrique de préférence).

. SOLUTIONS A DILUER :

Fluorure de benzalkonium 10%

Excipients : eau distillée ou purifiée, éthanol, antioxygène, glycérine, sorbitol, antiseptique. pH compris entre 4,5 et 6,5 (acide citrique de préférence).

APPLICATION DE L'INVENTION DANS LE DOMAINE DE L'ANTISEPTIE, DE L'ANTIBIOTHERAPIE ... ET NOTAMMENT DANS LA LUTTE CONTRE LES MST :

Les modes de réalisations préférentiels de l'invention par exemple pour son application en dermatologie et vénérologie à titre d'antiseptique sont les suivants :

. CREMES et LAITS :

Fluorure de benzalkonium 1,00%

Excipients : Eau distillée ou purifiée, humectants, émulsifiant, stabilisateur, antioxygène, antiseptique. (A répartir en proportions variables en fonction de la viscosité). pH de 4,5 à 6,5 (acide citrique de préférence).

. ONGUENTS et POMMADES :

Fluorure de benzalkonium 1,00%

Excipients : Eau distillée ou purifiée, émulsifiant, excipients du type corps gras (vaseline, lanoléine, lanovaseline, stéarovaseline), stabilisateur, anti-oxygène, antiseptique. (A répartir en proportions variables en fonction de la viscosité). pH de 4,5 à 6,5 (acide citrique de préférence).

. SAVONS SYNTHETIQUES EN PAINS OU EN PATES :

Fluorure de benzalkonium 1,8% à 2%

Excipients : Produits moussants et mouillants compatibles avec les ammoniums quaternaires (par exemple tensio-actif amphotère du type bétaine ou amino-bétaine) émoliants, stabilisateur, antioxygène, antiseptique régulateur du pH acide citrique.

. SOLUTIONS :

Fluorure de benzalkonium 1,00%

Excipients : Eau distillée ou purifiée, éthanol, antioxygène, glycérine, sorbitol, antiseptique. pH de 4,5 à 6,5 (acide citrique de préférence).

Les modes de réalisation préférentiels de l'invention, par exemple pour son application en dermatologie à titre d'antibiotique local sont les suivants :

. SOLUTIONS :

Erythromycine base 4%

Fluorure de benzalkonium de 0,2% à 0,8%

Excipients : Alcool éthylique, propylène glycol, eau distillée.

. GELS :

Erythromycine base 4%

Fluorure de benzalkonium de 0,2% à 0,8%

Excipients : Alcool éthylique, hydroxypropylcellulose, eau distillée, glycérine.

. POMMADES :

| | |
|---|---|
| Néomycine base | 0,35% |
| Fluorure de benzalkonium | de 0,2% à 0,8% |
| ou Bacitracine | 50 000 UI % |
| Fluorure de benzalkonium | de 0,2% à 0,8% |
| ou Oxytétracycline Chlorhydrate | 3% |
| Fluorure de benzalkonium | de 0,2% à 0,8% |
| ou Auréomycine Chlorhydrate | 3% |
| Fluorure de benzalkonium | de 0,2% à 0,8% |

Excipients : Vaseline, huile de vaseline, lanoline

. CREME :

Soframycine sulfate 2,5%

Fluorure de benzalkonium de 0,2% à 0,8%

Excipients : propylèneglycol, polyoxyéthylèneglycol, eau distillée.

Les modes de réalisation préférentiels de l'invention, par exemple pour son application en oto-rhino-laryngologie, à titre d'antibiotique local sont les suivants :

. POMMADES OPHTALMIQUES :

| | |
|---|---|
| Auréomycine | 1% |
| Fluorure de benzalkonium | de 0,2% à 0,8% |
| ou Oxytétracycline | 0,50% |
| Fluorure de benzalkonium | de 0,2% à 0,5% |

Excipients : Vaseline, huile de vaseline, lanoline.

. SOLUTION NASALE :

| | |
|---|---|
| Soframycine sulfate | 1,25% |
| Fluorure de benzalkonium | de 0,50% à 1% |

Excipients : Eau distillée, acide citrique, chlorure de sodium.

APPLICATION DE L'INVENTION NS LE DOMAINE DE LA DESINFECTION :

Ce domaine concerne le traitement des sols, surfaces, instruments ... par des produits bactéricides de contact.

Les modes de réalisation préférentiels de l'invention pour cette application sont les suivants :

| USAGE | FLUORURE DE BENZALKONIUM | EXCIPIENTS |
|---|---|---|
| Mains Epiderme | 0,05 à 0,1% | Eau purifiée ou alcool q. s. p. 100% |
| Instruments à stériliser à désinfecter | 0,08 à 1,0% | Eau purifiée q. s. p. 100% |
| Textiles | 0,05% | Eau purifiée q. s. p. 100% |
| Instruments type thermomètre | 0,081 à 1,0% | Ethanol 10% Eau purifiée q. s. p. 100% |
| Lavages des surfaces (chambres, planchers sols) | 0,1% | Eau purifiée q. s. p. 100% |

Les essais ont été menés suivant la norme AFNOR NET 72-150 mars 81 avec du fluorure de benzalkonium comme principe actif. Le neutralisant utilisé a été le suivant : 3% de Tween® 80 (V/V) et 0,3% de lécithine (M/V). Le pH du milieu étant de 7,2.

APPLICATION DE L'INVENTION EN COSMETOLOGIE :

Les modes de réalisation préférentiels de l'invention en cosmétologie sont décrits ci-après.

Les formes galéniques suivantes peuvent être présentées en cosmétologie: crèmes, laits, pommades, solutions, bains moussants, savons synthétiques, shampooings, lotions intimes, lotions désinfectantes.

Les formules des excipients sont les mêmes que pour les présentations pharmaceutiques mais les concentrations en Fluorure de benzalkonim seront différentes.

Ces concentrations sont les suivantes :

1,8% à 2% pour les produits rincés (type savons synthétiques)

0,1% à 0,2% pour les produits non rincés.

Peuvent également entrer dans ces formulations des principes actifs chimiques ou d'origine naturelle aux concentrations et doses autorisées en cosmétologie.

Les crèmes et laits peuvent être en phase continue aqueuse (émulsion huile/eau ou eau/huile) ou pâteuse à chaud se diluant dans l'eau.

Les différents excipients cités à titre d'exemple correspondent à titre indicatif non limitatif aux produits suivants :

. Humectants : glycérol, propylène glycol, diéthylène glycol, polyoxéthylène glycol.
. Emulsifiants : stéarate de sodium, cire d'abeilles, ester de sorbitol, ester de polyoxéthylène glycol, alcool gras, triethanolamine lanoline, tween, stéarate de glycol et polyglycols.
. Stabilisateurs : stéarate de glycol, alcool cétylique alginate, pectine, gomme, esters gras de polyols, esters de cellulose solubles.
. Antioxygènes : acide tartrique, citrique et ascorbique.
. Antiseptiques : acide borique, acide benzoïque, acide parabenzoïque et leurs esters méthyliques ou propyliques, sodés ou non.
. pH : toutes ces formulations sont particulièrement efficaces à des pH compris entre 4,5 et 6,5. pour obtenir cette fourchette on utilise principalement l'acide citrique.

**Revendications**

**1.** Application d'au moins un fluorure d'ammonium quaternaire purifié de formule générale :

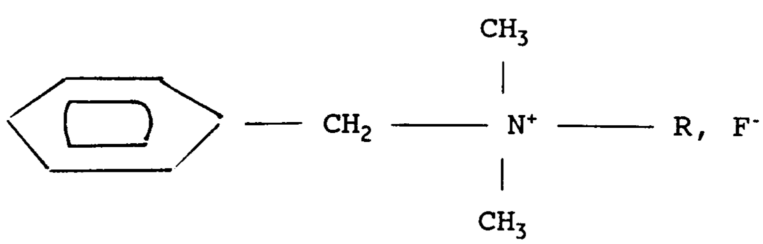

où R est un radical alcoyle pouvant varier entre $C_8\ H_{17}$ et $C_{18}\ H_{37}$ pour obtenir une composition destinée à inhiber ou à détruire au moins un virus ou un être vivant unicellulaire tel qu'un protozoaire, microbe, bactérie, gamète, champignon, levure ou autre en dehors de la sphère dentaire.

**2.** Application selon la revendication 1 pour obtenir une composition virucide.

**3.** Application selon la revendication 1 pour obtenir une composition fongicide.

**4.** Application selon la revendication 1 pour obtenir une composition pharmaceutique bactéricide destinée à être appliquée en dehors de la sphère dentaire.

**5.** Application selon la revendication 1 pour obtenir une composition destinée à être appliquée sur les organes génitaux pour lutter contre les maladies sexuellement transmissibles.

**6.** Application selon la revendication 1 pour obtenir une composition contraceptive locale, notamment spermicide.

**7.** Application selon la revendication 1 pour obtenir une composition cosmétique ou d'hygiène corporelle destinée à être appliquée en dehors de la sphère dentaire.

**8.** Application selon la revendication 1 pour obtenir une composition pour la désinfection locale du corps humain ou de surfaces en dehors de la sphère dentaire.

**9.** Application selon la revendication 1 pour obtenir une composition pharmaceutique antibiotique administrable par voie générale.

**10.** Application selon la revendication 1 pour obtenir une composition topique antibiotique.

**11.** Application selon la revendication 1 pour obtenir une composition topique destinée à lutter contre les affections virales, notamment les affections virales de la peau et des organes génitaux.

**12.** Application selon la revendication 1 pour obtenir une composition pharmaceutique administrable par voie générale ou parentérale destinée à lutter contre les virus ou les rétrovirus, notamment l'Herpès ou le LVA (ou HIV) responsable du SIDA.

**13.** Application selon la revendication 1 pour obtenir une composition destinée à lutter contre les affections mycosiques.

**14.** Application selon l'une quelconque des revendications 1 à 13 caractérisée en ce que R est compris entre $C_{12}H_{25}$ et $C_{14}H_{29}$.

**15.** Application selon l'une quelconque des revendications 1 à 14 caractérisée en ce qu'on utilise entre 0,05 % et 7 % en poids, notamment de l'ordre de 1 %, d'un tel sel d'ammonium quaternaire purifié.

**16.** Application selon l'une quelconque des revendications 1 à 15 caractérisée en ce qu'on utilise un autre principe actif choisi en fonction de la destination de la composition - notamment un antibiotique-, en combinaison avec le fluorure d'ammonium quaternaire.

**17.** Application selon l'une quelconque des revendications 1 à 16 caractérisée en ce qu'on utilise en outre au moins un fluorure métallique.

**18.** Application selon la revendication 17 caractérisée en ce qu'on utilise du fluorure de lithium.

**19.** Application selon la revendication 18 caractérisée en ce qu'on utilise en outre le conservateur Kathon (marque déposée), à base de 2-octyl-4-isothiazolin-3-one.

**20.** Utilisation d'un fluorure d'ammonium quaternaire selon la formule générale apparaissant à la revendication 1 à titre de contraceptif local, notamment de spermicide.

**21.** Composition inhibitrice ou destructrice d'au moins un virus ou d'au moins un être vivant unicellulaire tel qu'un protozoaire, microbe, bactérie, gamète, champignon, levure ou autre, caractérisée en ce qu'elle renferme au moins un fluorure d'ammonium quaternaire de formule :

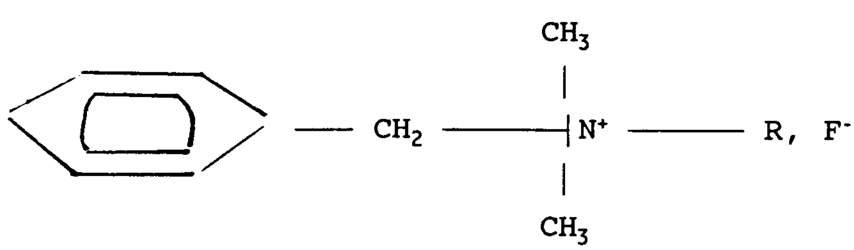

où R est un radical alcoyle pouvant varier entre $C_8H_{17}$ et $C_{18}H_{37}$ ainsi que du fluorure de lithium.

**22.** Composition selon la revendication 21 caractérisée en ce que R est compris entre $C_{12}H_{25}$ et $C_{14}H_{29}$.

**23.** Composition galénique selon l'une quelconque des revendications 20 à 22, caractérisée en ce qu'elle comporte entre 0,05 % et 7 % de poids, notamment de l'ordre de 1 %, d'un tel sel d'ammonium quaternaire suffisamment purifié de façon à pouvoir être utilisée pour l'être humain ou l'animal.

**24.** Composition antibiotique caractérisée en ce qu'elle renferme un principe antibiotique et un fluorure d'ammonium quaternaire de formule générale apparaissant à la revendication 1.

**25.** Composition selon la revendication 24 caractérisée en ce que R est compris entre $C_{12}H_{25}$ et $C_{14}H_{29}$.

## Claims

**1.** Application of at least one purified quaternary ammonium fluoride with the general formula of :

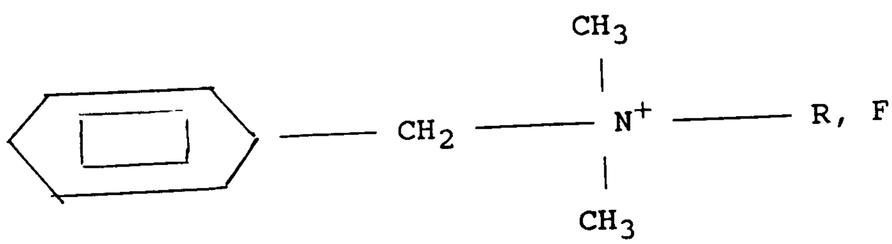

where R is an alkyl radical able to vary between $C_8$ $H_{17}$ and $C_{18}$ $H_{37}$ so as to obtain a composition for inhibiting or destroying at least one virus or a living unicellular organism, such as a protozoan, microbe, bacterium, gamete, mushroom, fungus or other substance outside the dental field.

**2.** Application according to claim 1 so as to obtain a virucide composition.

**3.** Application according to claim 1 so as to obtain a fungicide composition.

**4.** Application according to claim 1 so as to obtain a bactericide pharmaceutical composition to be applied outside the dental field.

**5.** Application according to claim 1 so as to obtain a composition able to be applied to genital organs to combat sexually transmissible diseases.

**6.** Application according to claim 1 so as to obtain a local contraceptive composition, especially a spermicide.

**7.** Application according to claim 1 so as to obtain a cosmetic or body hygiene composition to be applied outside the dental field.

**8.** Application according to claim 1 so as to obtain a composition for the local disinfection of the human body or surfaces outside the dental sphere.

**9.** Application according to claim 1 so as to obtain an antibiotic pharmaceutical composition able to be generally administered.

**10.** Application according to claim 1 so as to obtain a topical antibiotic composition.

**11.** Application according to claim 1 so as to obtain a topical composition for combatting viral infections, especially viral infections affecting the skin and the genital organs.

**12.** Application according to claim 1 so as to obtain a pharmaceutical composition able to be administered

generally or parenterally for combatting viruses or retroviruses, especially herpes or LVA (or HIV) responsible for AIDS.

**13.** Application according to claim 1 so as to obtain a composition for combatting mycotic infections.

**14.** Application according to any one of claims 1 to 13, wherein R is between $C_{12}H_{25}$ and $C_{14}H_{29}$ .

**15.** Application according to any one of claims 1 to 14, wherein between 0.05 and 7% in volume in weight and especially about 1% of such a purified quaternary ammonium salt is used.

**16.** Application according to any one of claims 1 to 15, wherein another active principle is used and selected according to the intended purpose of the composition - especially an antibiotic - combined with quaternary ammonium fluoride.

**17.** Application according to any one of claims 1 to 16, wherein at least one metallic fluoride is also used.

**18.** Application according to claim 17, wherein lithium fluoride is used.

**19.** Application according to claim 18, wherein a Z-octyl-4-isothiasdin-3-one based Kathon preservative (registered trademark) is used.

**20.** Use of a quaternary ammonium fluoride according to the general formula featured in claim 1 as a local contraceptive, especially as a spermacide.

**21.** Inhibiting or destructive composition of at least one virus or at least one unicellular living organism, such as a protozoan, microbe, bacterium, gamete, mushroom, fungus or other substance, wherein it contains at least one quaternary ammonium fluoride with the following formula :

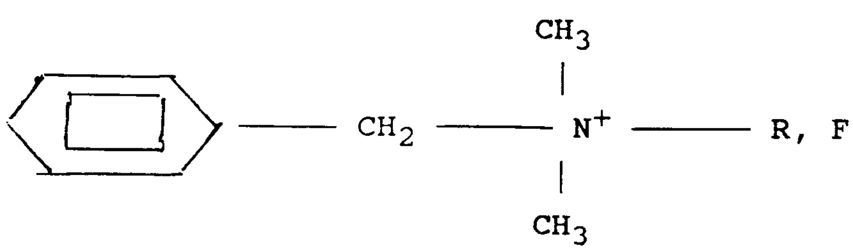

where R is an alkyl radical able to vary between $C_8H_{17}$ and $C_{18}H_{37}$ , as well as lithium fluoride.

**22.** Composition according to claim 21, wherein R is between $C_{12}H_{25}$ and $C_{14}H_{29}$ .

**23.** Galenic composition according to any one of claims 20 to 22, wherein it comprises between 0.05 and 7% in weight and in particular about 1% of such a quaternary ammonium salt sufficiently purified so as to be used for a human being or an animal.

**24.** Antibiotic composition, wherein it contains one antibiotic principle and one quaternary ammonium fluoride with the general formula featured in claim 1.

**25.** Composition according to claim 24 ,wherein R is between $C_{12}H_{25}$ and $C_{14}H_{29}$ .

**Patentansprüche**

**1.** Anwendung mindestens eines reinen quartären Ammoniumfluorids der allgemeinen Formel

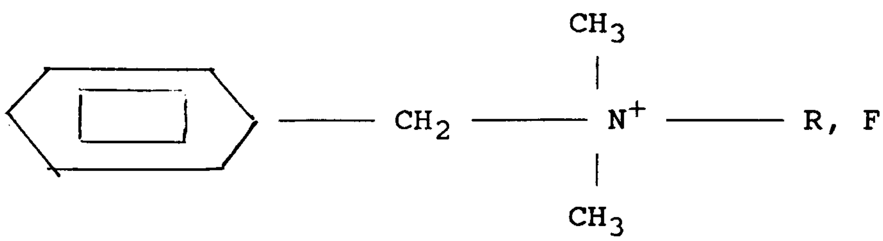

wobei R ein Alkylrest ist, der zwischen $C_8H_{17}$ und $C_{18}H_{37}$ schwanken kann, um eine Zusammensetzung zu erhalten, mit der mindestens ein Virus oder ein einzelliges Lebewesen von der Art Protozoen, Mikroben, Bakterien, Gameten, Pilze usw. außerhalb des Zahnbereichs gehemmt oder zerstört wird.

**2.** Anwendung nach Patentanspruch 1 zum Erhalten einer virentötenden Zusammensetzung.

**3.** Anwendung nach Patentanspruch 1 zum Erhalten einer pilztötenden Zusammensetzung.

**4.** Anwendung nach Patentanspruch 1 zum Erhalten einer pharmazeutischen bakterientötenden Zusammensetzung zur Anwendung außerhalb des Zahnbereichs.

**5.** Anwendung nach Patentanspruch 1 zum Erhalten einer Zusammensetzung für die Behandlung von Genitalien bei der Bekämpfung ansteckender Geschlechtskrankheiten.

**6.** Anwendung nach Patentanspruch 1 zum Erhalten einer örtlich empfängnisverhütenden, insbesondere spermtötenden Zusammensetzung.

**7.** Anwendung nach Patentanspruch 1 zum Erhalten einer kosmetischen oder körperhygienischen Zusammensetzung zur Anwendung außerhalb des Zahnbereichs.

**8.** Anwendung nach Patentanspruch 1 zum Erhalten einer Zusammensetzung für die örtliche Desinfektion des menschlichen Körpers oder Körperflächen außerhalb des Zahnbereichs.

**9.** Anwendung nach Patentanspruch 1 zum Erhalten einer pharmazeutischen antibiotischen Zusammensetzung zur oralen Einnahme.

**10.** Anwendung nach Patentanspruch 1 zum Erhalten einer örtlich antibiotischen Zusammensetzung.

**11.** Anwendung nach Patentanspruch 1 zum Erhalten einer Zusammensetzung für die örtliche Bekämpfung von Virenerkrankungen, insbesondere der Haut und der Genitalien.

**12.** Anwendung nach Patentanspruch 1 zum Erhalten einer pharmazeutischen Zusammensetzung zur oralen oder parenteralen Einnahme für die Bekämpfung von Viren und Retroviren, insbesondere der Gürtelrose- und der Aids-Erreger HIV.

**13.** Anwendung nach Patentanspruch 1 zum Erhalten einer Zusammensetzung für die Bekämpfung von Pilzerkrankungen.

**14.** Anwendung nach einem beliebigen der Patentansprüche 1 bis 13, dadurch gekennzeichnet, daß R zwischen $C_{12}H_{25}$ und $C_{14}H_{29}$ liegt.

**15.** Anwendung nach einem beliebigen der Patentansprüche 1 bis 14, dadurch gekennzeichnet, daß in einer entsprechenden Zusammensetzung zwischen 0,05 und 7 Gewichtsteile und insbesondere 1 Gewichtsteil von diesem reinen quartären Ammoniumsalz verwendet wird.

**16.** Anwendung nach einem beliebigen der Patentansprüche 1 bis 15, dadurch gekennzeichnet, daß je nach Bestimmung der Zusammensetzung und insbesondere bei einer antibiotischen Anwendung ein anderer Aktivstoff in Verbindung mit dem quartären Ammoniumfluorid verwendet wird.

**17.** Anwendung nach einem beliebigen der Patentansprüche 1 bis 16, dadurch gekennzeichnet, daß außerdem mindestens ein Metallfluorid verwendet wird.

**18.** Anwendung nach Patentanspruch 17, dadurch gekennzeichnet, daß Lithiumfluorid verwendet wird.

**19.** Anwendung nach Patentanspruch 18, dadurch gekennzeichnet, daß außerdem das auf der Grundlage von II-Octyl-IV-Isothiazolin-III-one bestehende Konservierungsmittel Kathon (eingetragenes Warenzeichen) verwendet wird.

**20.** Verwendung eines quartären Ammoniumfluorids nach der allgemeinen, in Patentanspruch 1 aufgeführten Formel als örtliches, insbesondere spermtötendes Empfängnisverhütungsmittel.

**21.** Zusammensetzung, mit der mindestens ein Virus oder ein einzelliges Lebewesen von der Art Protozoen, Mikroben, Bakterien, Gameten, Pilze usw. gehemmt bzw. getötet wird, dadurch gekennzeichnet, daß sie mindestens ein reines quartäres Ammoniumfluorid der allgemeinen Formel

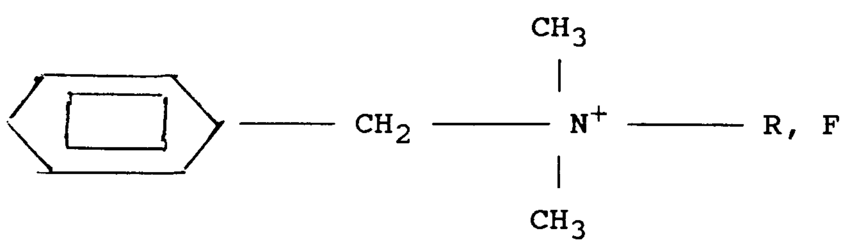

sowie Lithiumfluorid enthält, wobei R ein Alkylrest ist, der zwischen $C_8H_{17}$ und $C_{18}H_{37}$ schwanken kann.

**22.** Zusammensetzung nach Patentanspruch 21, dadurch gekennzeichnet, daß R zwischen $C_{12}H_{25}$ und $C_{14}H_{29}$ liegt.

**23.** Zusammensetzung nach einem beliebigen der Patentansprüche 20 bis 22, dadurch gekennzeichnet, daß sie zwischen 0,05 und 7 Gewichtsteile und insbesondere 1 Gewichtsteil von diesem quartären Ammoniumsalz enthält, das ausreichend gereinigt ist, um zur Behandlung von Menschen und Tieren verwendet werden zu können.

**24.** Antibiotische Zusammensetzung, dadurch gekennzeichnet, daß sie einen antibiotischen Wirkstoff und ein quartäres Ammoniumfluorid nach der allgemeinen, in Patentanspruch 1 aufgeführten Formel enthält.

**25.** Zusammensetzung nach Patentanspruch 24, dadurch gekennzeichnet, daß R zwischen $C_{12}H_{25}$ und $C_{14}H_{29}$ liegt.